# EUROPEAN PATENT APPLICATION

(11) **EP 4 029 456 A1**
(43) Date of publication of application: **20.07.2022**
(21) Application number: 21151528.3
(22) Date of filing: 14.01.2021
(51) Int. Cl.: A61B 10/00, A61B 10/02

(54) **A STICK FOR COLLECTING AND TRANSFERRING BIOLOGICAL MATERIAL**

(71) Applicant: Albireo Biomedical sp. z o.o., 40-057 Katowice (PL)
(72) Inventor: Kaszynski, Grzegorz, 01-892 Warszawa (PL); Glowacki, Maciej, 04-087 Warszawa (PL)
(74) Representative: Wroblewski, Marcin Jan

(57) **Abstract**

A stick (1) for collecting and transferring biological material, in particular a fluid containing genetic material, comprising a handle (2) connected to a head (3), whose body (5) comprises two parts (6), (7) whose back walls are adjacent to each other, adapted to collecting and storing biological material, characterised in that at least one of the parts (6), (7) comprises at least one space (9) constituting a retention reservoir for the biological material and at least two protrusions (10) defining the channels for feeding the collected biological material into the space (9), wherein said protrusions (10) have a shape similar to the letters V or C or U, whose arms face the proximal end of the stick (1).

## Description

### Field of the invention

The invention relates to a stick for collecting and transferring biological material, in particular a fluid containing genetic material.

### Prior art description

The primary task of the swab stick is to collect the maximum possible amount of fluids containing genetic material, such as virus genetic material, as conveniently and safely as possible.

US patent application US20190261962A1 discloses a device for collecting biological samples from a patient, for example from their oesophagus. Said device comprises a part for collecting biological samples and an elongated support member, which may be tubular in shape. One end of the support member is connected to the part for sample collection, and the other end, for example, to a syringe for introducing or collecting fluids. Said part for collecting biological material, especially in the form of liquid, is made of a flexible polymer, such as silicone or polyurethane. Rib-shaped projections arranged across the surface thereof form channels that discharge biological material into cups defined by arms of the protrusions arranged in a V-shape. Said ribs are located substantially perpendicular to the longitudinal axis of the device, i.e. at an angle of approximately 90°. The outline of the cups, in turn, is trapezoidal in cross-section.

European patent EP2741675B1 discloses a device for collecting and transferring swabs in the form of a stick containing a hollow rod, which constitutes the bearing body of the device. One of its end comprises a part for collecting swabs in the form of biological material, while the opposite end is adapted for coupling to the tubular suction member of the collection unit. Said collection unit is adapted to be gripped, and thus for gripping the swabbing device. The part for collecting swabs comprises a chamber and transverse tubes which are channels for directing the swab into the inner space of said chamber, which is followed by the collected biological material being transported from said chamber through a tubular member to the collection unit. Alternatively, the part for collecting swabs may be a spongy member comprising a plurality of channels for transporting the swab upon collection.

American patent US2017065261A1 discloses a device for collecting and transferring swabs in the form of a stick containing a support body. The support body comprises a part for collecting swabs part on one side and a gripping part on the other side. The diameter of said support body may decrease towards the part for collecting biological material. Said part for collecting swabs also comprises protrusions that form channels arranged at an angle between 45° and 75° to the longitudinal axis of the stick. Furthermore, the longitudinal body and the part with protrusions for swabbing form an integral whole. The disclosed stick for collecting and transferring swabs may be made of polypropylene or high impact polystyrene. Furthermore, between the gripping part and the collecting part of said stick, a weakening part is located designed for breaking said stick.

Also, US patent application US2006240413A1 discloses a monolithic probe in the form of a stick for collecting samples of biological material, in particular for collecting vaginal biological material. Said probe comprises a support body having a gripping part and a part for collecting biological material, located on opposite sides of the body. The part for collecting biological material comprises spaces for the collection of the latter and channels arranged perpendicular to the axis of symmetry of the probe.

The object of the invention is to provide a stick for collecting and transferring biological material, in particular a fluid containing genetic material, allowing for increasing the amount of biological material collected during swabbing.

### Essence of the invention

A stick for collecting and transferring biological material, in particular a fluid containing genetic material, comprising a handle connected to a head, whose body comprises two parts, whose back walls are adjacent to each other, adapted to collecting and storing biological material, characterised in that at least one of the parts adapted for collecting and storing biological material comprises at least one space constituting a retention reservoir for the biological material and at least two protrusions defining the channels for feeding the collected biological material into the space, wherein said protrusions have a shape similar to the letters V or C or U, whose arms face the proximal end of the stick.

The design of the stick according to the invention provides an increase in the level (efficiency) of collection of biological material during swabbing.

In particular, directing the arms of the protrusions having a shape similar to the letter V towards the proximal end of the stick results in that during its longitudinal movement, for example as it is pulled out of the nostril of the person being swabbed, more biological material is collected and directed to the spaces forming the retention reservoirs.

Preferably, the arms of the protrusions having the shape similar to the letter V are arranged relative to the longitudinal axis of the stick at an angle between 45° and 90°, most preferably 60°. The inclination of the arms of the protrusions at an angle from the indicated range ensures a reduced discomfort for the person from whom the biological material is being collected. This is particularly relevant for protrusions with a shape similar to the letter V.

Preferably, the stick comprises scraping protrusions in the form of ribs located between the protrusions that delineate the channels for supplying biological material to the reservoirs. The use of said protrusions reinforces the structure of the stick. Moreover, the scraping protrusions ensure a further increase in the amount of biological material collected.

Preferably, the scraping protrusions are located at an equal distance from the protrusions defining the channels for supplying the biological material to the retention reservoirs. The disclosed location of the scraping protrusions and of the protrusions defining the channels optimises the transfer of the biological material collected from the said channels to the spaces constituting the retention reservoirs.

The protrusions defining the channels that supply the biological material to the retention reservoirs and the scraping protrusions are preferably trapezoidal in cross-section with a larger bottom base.

Preferably, the stick according to the present invention comprises a homogeneous core between the parts adapted for collecting and storing biological material. The use of a homogeneous core reduces the risk of breaking the stick, while maintaining the greatest possible depth of the retention reservoirs and supply channels.

Preferably, the walls of the space constituting the retention reservoir are inwardly inclined at an angle from 5° to 30°, preferably approximately 15°. The inward inclination of the walls of the space at an angle ranging from 5° to 30° prevents the formation of air pockets, which reduce the volume of the swab collected, thus increasing the efficiency of collecting biological material.

Preferably, the space constituting the retention reservoir has the form of a cone.

Preferably, the spaces are evenly distributed across the X axis of the stick, wherein the longitudinal X axis of the stick is preferably the axis of symmetry of the stick. This arrangement allows for applying the maximum possible depth of the spaces without damaging the stick and for evenly distributing the biological material collected across both parts of the body of the head.

Preferably, the spaces are arranged centrally across the axis of symmetry of the stick. Again, this arrangement allows for applying the maximum possible depth of the spaces without damaging the stick and for evenly distributing the biological material collected across both parts of the body of the head.

Preferably, the two parts adapted for collecting and storing the biological material comprise at least one space constituting a retention reservoir and at least two protrusions defining channels for supplying the biological material into said retention reservoir.

Preferably, the diameter of the stick decreases towards the body of its head.

Preferably, the stick is a monolithic part made entirely of a single material.

Preferably, the stick is made of polypropylene, polystyrene, polyester, methyl butadiene-styrene methacrylate copolymer (MBS resin), polyamide, polycarbonate, polyvinyl chloride, polymethyl methacrylate, acrylonitrile-butadiene-styrene, or a mixture of said materials.

Preferably, at least one weakening part for breaking the stick is located between the handle and the head.

### Advantages of the invention

The design of the stick according to the invention allows for conveniently collecting a large amount of biological material. The large number of small channels results in a large contact surface between the stick and the mucosa, which reduces the feeling of discomfort. The fact that the entire stick is made of a single material as a monolithic unit, using a single injection process, reduces production costs, minimises the risk of toxicity and the risk of poisoning in the molecular reaction during the test (e.g. following a contact of the mucosa with an adhesive and/or flock). Furthermore, the stick according to the invention allows for simplifying the process of scaling up the production thereof, since a single machine is required to manufacture it.

Moreover, the stick provided as a monolithic unit both simplifies the process of its certification and facilitates the process of its sterilisation.

The design of the stick according to the invention provides an increase in the amount of biological material filled into vials for storing and/or transferring samples relative to the amount of biological material collected. Now, prior art solutions, such as flocked sticks, for example, involve a large amount of the biological material collected remaining on the surface of the flock.

### Description of the figures of the drawing

The object of the invention is shown in the embodiments in the drawing where:
- Fig. 1: is a front view of the stick according to the invention;

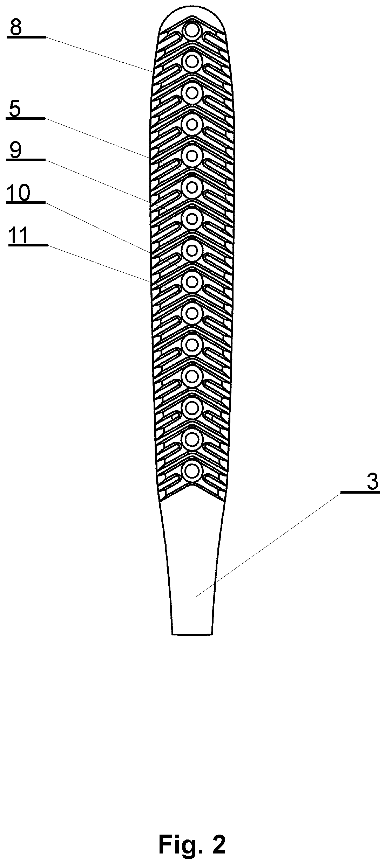

- Fig. 2: is a front view of a head of the stick according to the invention;
- Fig. 3a - 3c: are protrusions arranged in a shape similar to the letters V, U and C.

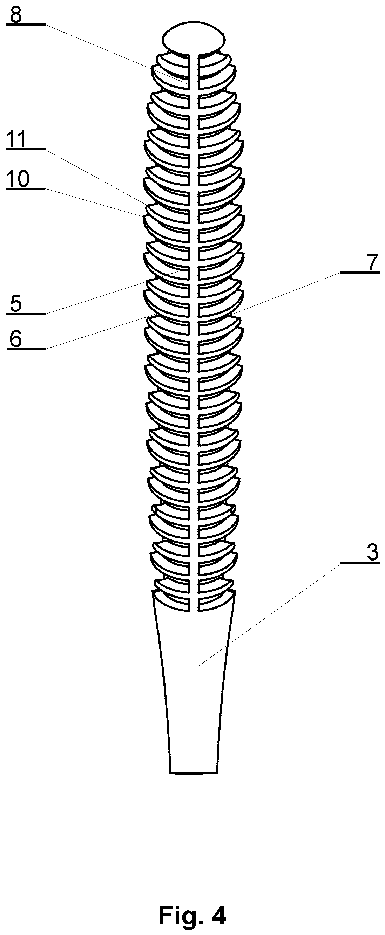

- Fig. 4: is a side view of the head of the stick according to the invention;

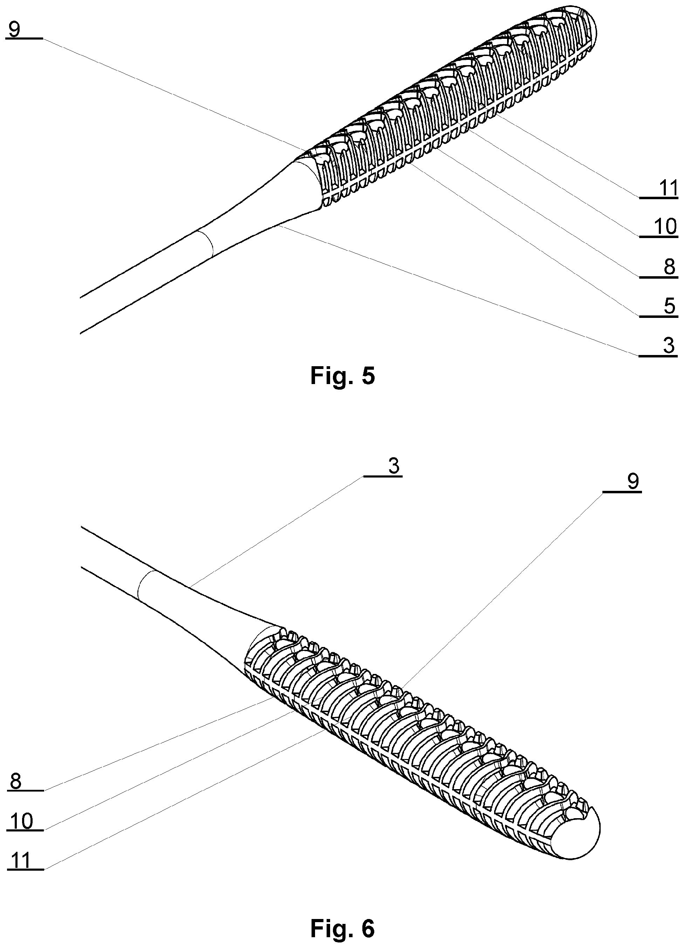

- Fig. 5, 6: are a perspective view of the head of the stick according to the invention.

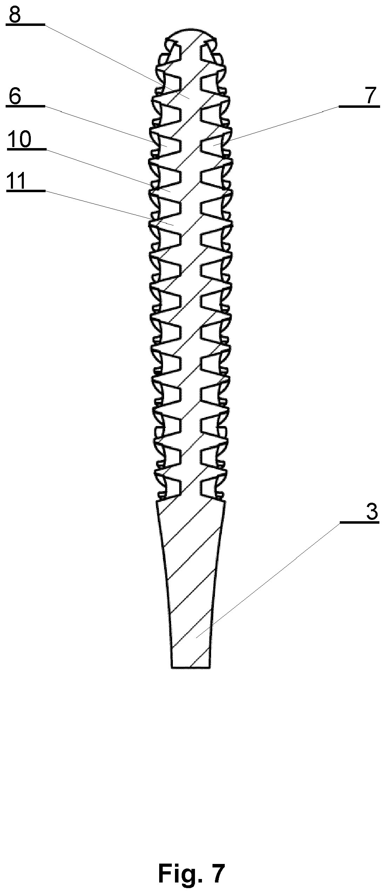

- Fig. 7: is a sectional view of the head of the stick according to the invention along its longitudinal axis.

### Detailed Description of the Invention

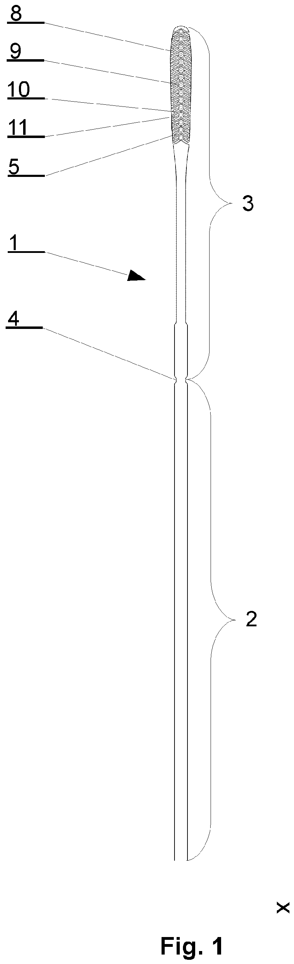

Fig. 1 is a stick 1 for collection and transfer of biological material, in particular a fluid containing genetic material. For example, the stick 1 may be used during swab collection when testing for a virus, such as SARS-CoV-2. The stick 1 may also be used during the collection of biological material for genetic testing.

The stick 1 comprises a handle 2 connected to a head 3 for collecting biological material. Between the handle 2 and the head 3 there is a weakening part 4 for breaking the stick 1 in the form of a circumferential recess in the handle 2, which alters the diameter of the handle 2 at this point from, for example, 2.5 mm to 1.8 mm or even 1.5 mm. The end of the handle 2 situated at the end of the stick 1 opposite to the head 3 is the proximal end of the stick 1. A smaller diameter of the handle 2 in the weakening part 4 could cause the stick 1 to break due to excessive tension during the collection of biological material (swabbing). The weakening part 4 is situated at a height suitable for a particular vial model for sample storage and/or transport. If more weakening parts 4 are used, the stick becomes more versatile as it can be used in various models of vials designed for storing and/or transferring samples. Furthermore, the diameter of the handle 2 decreases towards the body 5 of the head 3, for example from 2.5 mm to 1.8 mm.

The shape of the head 3 of the stick 1 is selected based on the intended use so as to optimise the collection of the biological material by providing the largest possible area of contact between the body 5 of the head 3 and the surface containing said biological material. For example, the body 5 of the head 3 may have an elongated shape so as to allow its insertion into the nose in a minimally invasive manner. Alternatively, the head 2 of the stick 1 may have a barrel shape so as to optimise the collection of biological material from the pharynx, in particular from the tonsil area.

In the present example, the body 5 of the head 3 comprises two parts 6, 7 adapted for collecting and storing biological material that are separated from each other by a core 8 arranged along the longitudinal axis of the stick 1. The use of the core 8 between the parts 6, 7 minimises the risk of the stick 1 breaking at the height of the body 5 of the head 3 during swabbing.

The two parts 6, 7, adapted for collecting and storing biological material, comprise spaces 9 that constitute retention reservoirs for biological material, thus allowing for significantly increasing the volume of biological material collected. Preferably, the spaces 9 have a diameter at the inlet of approximately 1 mm, which does not structurally weaken the body 5 of the head 3. The depth of the spaces 9, in turn, varies from 0.8 to 1.5 mm, depending on the position of a particular space 9. The shape of the spaces 9 can be conical, for example frustoconical, with the walls inclined inwardly at an angle of approximately 15° (other embodiments use different angles ranging from 5° to 30°). This inclination of the walls prevents the formation of air pockets, which reduce the volume of the spaces 9 (retention reservoirs) and thus reduce the amount of swab collected. Elimination of the formation of such air pockets results in increased efficiency of biological material collection. In the present embodiment, said spaces 9 are distributed evenly across the axis of symmetry of the stick 1. This allows for applying the maximum possible depth of the spaces 9 without damaging the stick 1 and for evenly distributing the biological material collected across both parts 6, 7 of the body 5 of the head 3.

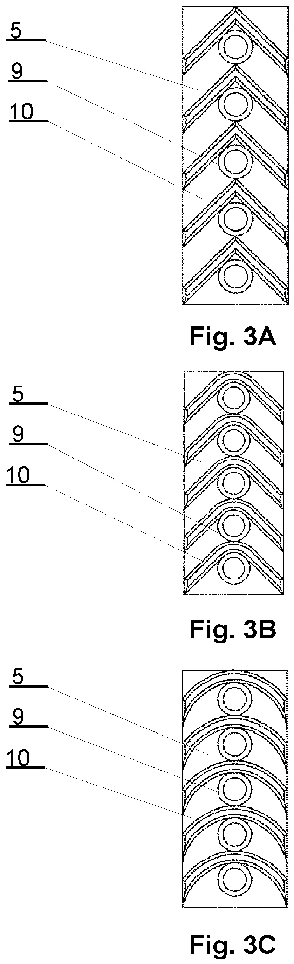

Both parts 6, 7 of the body 5 also contain rigid protrusions 10 defining the channels for supplying the biological material collected into the spaces 9 (retention vessels). The individual channels cover the space between adjacent protrusions 10, with the width of the individual channels preferably identical. The individual protrusions 10 have a shape similar to the letter V, with its the arms facing the proximal end of the stick 1. In cross-section, the shape of the protrusions 10 is trapezoidal shape with a larger bottom base. In other examples, the protrusions have a shape similar to the letter U or C as shown in Fig. 3B and Fig. 3C. The arrangement of the protrusions 10 as described above allows for directing a greater amount of the biological material collected to the spaces 9, especially when the stick 1 is being taken out from the nostril of the person undergoing the swabbing procedure. This increases the amount of genetic material collected during swabbing. The protrusions 10 may have a height of approximately 0.4 mm. The arms of said protrusions 10 are arranged relative to the longitudinal axis of the stick 1 at an angle of 45° to 90°, most preferably at an angle of 60°, which significantly reduces the discomfort of the person from whom the biological material is being collected. At smaller angles of inclination of the arms of the protrusion 10 relative to the longitudinal axis of the stick 1, the connection points of these arms (the tips of the protrusions) are excessively pointed, which causes a risk of irritation and discomfort for the person undergoing the swabbing procedure.

Furthermore, the stick 1 comprises additional scraping protrusions 11 in the form of ribs located between the protrusions 10 having a shape similar to the letter V. Said scraping protrusions 11 provide reinforcement to the structure of the stick 1. During the rotation of the stick 1, said protrusions additionally collect the collected biological material into the channels defining by the protrusions 10, which promotes a further increase in the amount of the material collected. Said scraping protrusions 11 are preferably located at an equal distance from the protrusions 10. This optimises the transfer of the biological material collected from the channels defining by the protrusions 10 to the spaces 9 (retention reservoirs).

In addition, the scraping protrusions 11 have a trapezoidal shape in cross-section with a larger bottom base.

The stick 1 is a monolithic component made entirely of a single material as a result of a single injection moulding process, such as polypropylene, polystyrene, polyester, methyl butadiene-styrene methacrylate copolymer (MBS resin), polyamide, polycarbonate, polyvinyl chloride, polymethyl methacrylate, acrylonitrile-butadiene-styrene, or a mixture of said materials. The stick 1 may also be made of materials that are mixtures of said materials.

The stick 1 may be used for collecting and transferring biological material e.g. from the nasopharynx and pharynx.

## Claims

1. A stick (1) for collecting and transferring biological material, in particular a fluid containing genetic material, comprising a handle (2) connected to a head (3), whose body (5) comprises two parts (6), (7) whose back walls are adjacent to each other, adapted to collecting and storing biological material, **characterised in that** at least one of the parts (6), (7) comprises at least one space (9) constituting a retention reservoir for the biological material and at least two protrusions (10) defining the channels for feeding the collected biological material into the space (9), wherein said protrusions (10) have a shape similar to the letters V or C or U, whose arms face the proximal end of the stick (1).

2. The stick according to claim 1 **characterised in that** the arms of the protrusions (10) having the shape similar to the letter V are arranged relative to the longitudinal axis of the stick (1) at an angle between 45° and 90°.

3. The stick according to claim 1 or 2 **characterised in that** it comprises scraping protrusions (11) in the form of ribs arranged between the protrusions (10).

4. The stick according to claim 3 **characterised in that** the scraping protrusions(11) are arranged at an equal distance from the protrusions (10).

5. The stick according to any one of the preceding claims, **characterised in that** it comprises a homogeneous core (8) between the parts (6), (7).

6. The stick according to any one of the preceding claims, **characterised in that** the walls of the space (9) are inwardly inclined at an angle between 5° and 30°.

7. The stick according to any one of the preceding claims, **characterised in that** the space (9) is in the form of a cone.

8. The stick according to any one of the preceding claims, **characterised in that** the spaces (9) are evenly distributed across the X axis of the stick (1).

9. The stick according to claim 6 **characterised in that** the X axis of the stick (1) is the axis of symmetry of the stick (1).

10. The stick according to any one of the preceding claims, **characterised in that** the spaces (9) are arranged in the axis of symmetry of the stick (1).

11. The stick according to any one of the preceding claims, **characterised in that** both parts (6), (7) comprise at least one space (9) and at least two protrusions (10).

12. The stick according to any one of the preceding claims, **characterised in that** the diameter of the stick (1) decreases towards the body (5) of the head (3).

13. The stick according to any one of the preceding claims, **characterised in that** it is a monolithic part made entirely of one material.

14. Preferably, the stick according to any one of the preceding claims is made of polypropylene, polystyrene, polyester, methyl butadiene-styrene methacrylate copolymer (MBS resin), polyamide, polycarbonate, polyvinyl chloride, polymethyl methacrylate, acrylonitrile-butadiene-styrene, or a mixture of said materials.

15. The stick according to any one of the preceding claims, **characterised in that** between the handle (2) and the head (3) there is at least one weakening part (4) for breaking the stick (1).
